# EUROPEAN PATENT APPLICATION

(11) **EP 2 592 431 A1**
(43) Date of publication of application: **15.05.2013**
(21) Application number: 11188849.1
(22) Date of filing: 11.11.2011
(51) Int. Cl.: G01R 33/48, G01R 33/563

(54) **Method and apparatus for visualization of tissue perfusion by means of assessing BOLD signal fluctuations**

(71) Applicant: Max-Planck-Gesellschaft zur Förderung der Wissenschaften e.V., 80539 München (DE); Virginia Polytechnic Institute and State University, Blacksburg, VA 24061 (US)
(72) Inventor: Villinger, Arno, 14195 Berlin (DE); Margulies, Daniel, 10999 Berlin (DE); Lv, Yating, 04105 Leipzig (DE); Craddock, Richard Cameron, Hoboken New Jersey 07030 (US)
(74) Representative: Hannke, Christian

(57) **Abstract**

The invention relates to method, an apparatus and the use thereof of visualization of areas of the tissue insufficiently supplied with blood, in particular in the event of an acute stroke, by evaluation of local fluctuations of a BOLD signal comprising the following steps:
- producing and recording of BOLD signal data by means of an apparatus (8);
- passing on the BOLD signal data to a data detection device (10) by way of at least one interface;
- sending the BOLD signal data received from the data detection device (10) to an evaluation device;
- pre-processing BOLD in a first evaluation device (12) for image recognition;
- applying a time delay analysis to the BOLD signal data of at least one voxel in a second evaluation device (14) in order to calculate a time dependent positive and negative phase shift (I1, 12) of the BOLD signal data of at least one voxel, and
- assigning a reference value which reference value is an estimated time delay between the BOLD signal data of at least one voxel and the entire average time course of a selected region during the recording of the BOLD data in order to estimate a modified BOLD signal data which are used to determine at least one area of the hypoperfused tissue.

## Description

The present invention relates to a method, an apparatus and the use thereof for visualization and identification of hypoperfused tissue areas by assessing the time delays of the spontaneous local fluctuations of a blood-oxygen-level-dependence (BOLD) signal. Moreover, the present invention may be used to identify in the brain an area of perfusion deficit, e.g., in the event of a stroke.

There are several mechanisms for measuring BOLD signal, for example but not limited to, functional magnetic resonance imaging (fMRI) and functional near-infrared spectroscopy (fNIRS). For illustration, we describe the novel method of visualization and identification of hypoperfused tissue areas by employing fMRI as an example. However, it should be understood that the method according to the invention may be applied using fNIRS, or any other method that measures the ratio of oxygenated haemoglobin to deoxygenated haemoglobin, and is not restricted to the present example.

Functional magnetic resonance imaging (fMRI) may be used to measure the BOLD signal (e.g., haemodynamic response and/or change in blood flow) related to neural activity in the brain or spinal cord. Active areas of the brain generally require more oxygen, which causes an intensity change in the BOLD signal. In fMRI, the oxygenation state of haemoglobin influences the intensity of an MRI signal. For example, in oxygenated blood, oxygen molecules are bound to haemoglobin's iron atoms, shielding the effect that iron otherwise has on magnetic resonance imaging (MRI) signal. Oxygenated haemoglobin contained in the blood is thus characterized as diamagnetic. De-oxygenated haemoglobin, lacking the shielding oxygen molecules, has paramagnetic properties. The MRI signal is thus a blood-oxygen-level-dependent (BOLD) signal that allows identification and visualization of active areas of the brain.

In the event of a stroke the blood flow to the tissues of the brain may be disrupted at least in part, for example by vascular damage or blocking. The blood flow to the tissues of the brain may be described by the perfusion rate, which is the volume of blood that passes through a mass of tissue per a unit of time. Interruption of blood flow may create a perfusion deficiency. A perfusion deficiency may reduce the amount of oxygen available to tissues, cells, and neurons, of the brain.

Perfusion deficiencies (for example, hypoperfusion) may be assessed in terms the time it takes for blood to travel from one region of the brain to another region of the brain. This measurement may be referred to as mean transit time (MTT). Currently known MRI methods have been unable to detect perfusion deficiencies without the use of contrast agents such as gadolinium.

Health care professionals rely upon detection mechanisms to locate and diagnosis areas of hypoperfusion. Diagnoses and location of hypoperfused areas allows health care professionals to administer suitable therapeutic approaches to re-establish the supply of blood to the brain, such as for example a thrombolysis treatment, may dissolve existing blood clots and correct the hypoperfusion which occurs. If this is not possible due to time-consuming examinations of the patient's brain, the risk of necrosis arises, so that the tissue with the inadequate blood supply dies irreversibly and permanent damage results.

Currently, health care providers must rely upon contrast-based diagnostic methods. Contrast-based methods are not desired for at least two reasons. The use of contrast agents represents at least one invasive step because these agents must be injected into the patient's blood. Secondly, the contrast agents carry serious health risks. The risks of using contrast agents also include, for example but not limited to, severe side-effects (e.g., in patients with kidney disease), and the inability to repeat examinations for monitoring purposes.

The prevalent method of identifying hypoperfused areas in tissue, e.g. pathophysiological information of patients with an acute stroke, is to use a combination of perfusion-weighted imaging and diffusion-weighted imaging. The seriously damaged centre of the stroke, also called infract core, can be illustrated by means of the diffusion-weighted imaging (DWI). The perfusion-weighted imaging (PWI) (with controversial contrast enhancement) may allow illustration and calculation of the hypoperfused area of the brain. This area may include portions of the infarct core, as well as tissue at risk for permanent damage.

Specifically, perfusion and diffusion MRI (perfusion-weighted imaging: PWI; diffusion-weighted imaging: DWI) are employed in clinics as well as in research to identify pathophysiological patterns in patients with, for example but not limited to, acute stroke and similar disorders. DWI is thought to roughly reflect the severely damaged infarct core, and PWI reflects the area of hypoperfusion. The volumetric difference between these two (PWI- minus DWI-volume) is also termed the PWI/DWI-mismatch, and has been suggested to be a MRI surrogate of the 'ischemic penumbra'. The ischemic penumbra represents preferably the area with a reduction in cerebral blood flow (CBF), resulting in impaired neuronal function but preserved brain structure. If CBF is restored in time, the ischemic damage in the penumbra can be reversed, and therefore the penumbra is regarded as the prime target for any treatment approach.

A penumbra may be understood in this case as being the hypoperfused tissue surrounding the infarct core. In case of a restored cerebral blood flow in time, the ischaemic damage of this penumbra area is reversible. In this case the infarct core may be understood as being the area of the tissue of the clogging of the arteries or constriction of the blood vessels, for example.

For example, if the PWI and DWI volumes determined correspond to each other or if the DWI volume predominates, then it may be assumed that there is no further risk of an increase or a spread of the stroke. If, on the other hand, the PWI volume is significantly larger than the DWI volume, a spread of the area of the stroke in the brain may be assumed, so that in some cases the hypoperfused area will also become necrotic as a result of the stroke.

In addition, it is possible that within 12 hours after the acute stroke, a recanalization will take place, for example by an artery clogged by a blood clot being freed again by treatment with medication. The perfusion of the affected brain, if the area is located and determined, may be treated as discussed above so as to prevent a spreading of the stroke.

The mismatch concept for identifying the penumbra has been challenged as being frequently inaccurate due to the lack of good quantification of perfusion at low perfusion levels and its susceptibility to alterations of the arterial input. Another disadvantage is the necessity of contrast agent application for perfusion MRI. Only when the cerebral blood flow in the brain is re-established as quickly as possible, the ischaemic damage will be reversible. In addition, it is known that the contrast agents used may cause allergies which have a particularly adverse effect upon the state of the body in the case of stroke patients.

Thus, one object of the disclosed method, apparatus and use thereof is to provide a non-invasive, simple, rapid measure of tissue perfusion, for example, towards diagnosis of possible spreading of an acute stroke in the brain. Moreover, a further objective includes eliminating the use of contrast agents. An additional objective of the present invention is to provide a less toxic, non-invasive, and more robust method, apparatus and use thereof of identifying and diagnosing hypoperfused brain regions which may be integrated into existing systems and equipment such as but not limited to MRI, fNIRS, or any other apparatus capable of measuring the ratio of the concentration of oxygenated haemoglobin to deoxygenated haemoglobin.

This is made possible according to the disclosure with the method in claim 1, the apparatus in claim 7 and the use in claim 9.

Further advantageous variations are the subject matter of the sub-claims.

The concept of the method of the present invention reveals in the following methodological steps for a method of visualization of areas of the tissue insufficiently supplied with blood, in particular in the event of an acute stroke, by evaluation of local fluctuations of a blood-oxygen-level-dependence (BOLD) signal comprising:
- producing and recording of BOLD signal data by means of an appropriate apparatus;
- passing on the BOLD signal data to a data detection device by way of at least one interface;
- sending the BOLD signal data received from the data detection device to an evaluation device;
- pre-processing the BOLD signal data in a first evaluation device;
- applying a time delay analysis to the BOLD signal data of at least one voxel in a second evaluation device (14) in order to calculate a time dependent positive and negative phase shift (I1, 12) of the BOLD signal data of at least one voxel, and
- assigning a reference value which reference value is an estimated time delay between the BOLD signal data of at least one voxel and the entire average time course of a selected region during the recording of the BOLD data in order to estimate a modified BOLD signal data which are used to determine at least one area of the hypoperfused tissue.

In one variation, the BOLD signal of the brain activity of a patient who is suspected of having had an acute stroke may be initially produced and recorded by means of an fMRI scanning apparatus. The data production and recording may be carried out by means of conventional fMRI scanners. It may be advantageous for the disclosed method to be carried out by means of fMRI data acquired independent of any specific requirements in order to facilitate data acquisition, for example in patients. Moreover, for data production and recording also other techniques like fNIRS or similar measuring the ratio of the concentration of oxygenated haemoglobin to deoxygenated haemoglobin may be used for the method according to the invention.

In a further step, the BOLD image, which may be magnetic resonance imaging base signal data, produced and recorded are passed on to a data detection device, for example by way of at least one interface which can be designed for example in the form of a hardware or a network interface. Furthermore, it would be possible for the interface to be present in parallel or in series. In addition, it is possible for a plurality of interfaces of this type to be present. The data detection device may be used for junction of all data detected and may permit a temporary storage of the BOLD signal data or even storage of the BOLD signal data. Furthermore, the data detection device may send the data received from the scanning apparatus, for example, in the form of data packets, on to an evaluation device, or may be integrated such that the data detection device also performs the evaluation function.

Furthermore, a first evaluation device may actively retrieve the BOLD signal, which may be magnetic resonance imaging base signal data, detected and recorded by the data detection device from the data detection device, so that the data detection device can be designed in the form of both a receiving and a transmitting device.

The BOLD signal, which may be magnetic resonance imaging base signal data, produced and recorded in method steps may optionally be pre-processed in a first evaluation device for image recognition improvement of the quality of the BOLD signal data, for example. This pre-processing of the BOLD signal data may be applied to all the BOLD signal data which are detected during an examination, so that all the pre-processed data are advantageously capable of being ordered in a standardized x-y-z coordinate system reproducing the corresponding area of the brain in a reliable manner, e.g. substantially without error deviation, in a subsequent three-dimensional reconstruction.

As a next step, a time delay analysis to the BOLD signal data of at least one voxel in a second evaluation device is applied in order to calculate a time dependent positive and negative phase shift of the bold signal data of at least one voxel and finally a reference value is assigned.

In the foregoing the term "voxel" refers to a volumetric, three-dimensional pixel as a discrete unit of volume.

By applying an analysis to the BOLD signal data of at least one voxel in, for example but not limited to, the image reconstruction region of an MRI scanner, the first evaluation device, a second evaluation device, or otherwise, a calculation of an alteration of the time-dependent information is realized, for example: allocating to each voxel a value which is related to the phase shift between the voxel's time course and a reference.

This may be achieved by, for example, measuring the time delay between two BOLD signals, which may be preferably achieved by obtaining a voxel reference value, e.g., by extracting a per voxel mean time course of BOLD activity from a region of the brain, obtaining the time course of every voxel in the brain and assign a value to each voxel, which value is an estimate of that voxel's delay, e.g., the relationship between the time course of the specific voxel and the reference value (e.g., the mean time course of BOLD activity of a reference region), assigning each voxel an identifier (e.g., a marker like a color) which represents that voxel's assigned estimated time course delay and integrating the voxel identifiers to create an image, for example, a visual map of the patient's brain.

The estimate of each voxel's time delay may be determined by using, for example but not limited to, time-shifted correlation between the voxel's time course and the reference value, time-shifted covariance analysis of the voxel's time course and the reference value, time-shifted mutual information analysis of the voxel's time course and the reference value, and Fourier transforms of the voxel's time course and the reference value and then estimating the difference between the phase values.

Moreover, in a further variation, the BOLD signal data, which may be magnetic resonance imaging base signal data pre-processed in the first evaluation device may be sent to a second evaluation device or may be retrieved from the second evaluation device. For example, in the second evaluation device, the data may assessed for tissue changes in the time-dependent activity, for example, through an analysis of the phase shift of the pre-processed BOLD signal data, wherein a time dependent positive and negative phase shift of the time signal course of the BOLD signal data of at least one voxel, alternatively a multiplicity of voxels, or alternatively all the voxels of a defined region, takes place. This of course is only to be regarded as one variation. It would also be possible for both the pre-processing and the application of the time shift analysis to be carried out by a single evaluation device. It is preferable for the time delay analysis to be designed in the form of an algorithm.

In a further method step, each voxel's time delay may be estimated by using, for example but not limited to, time-shifted correlation between the voxel's time course and the reference value, time-shifted covariance analysis of the voxel's time course and the reference value, time-shifted mutual information analysis of the voxel's time course and the reference value, and Fourier transforms of the voxel's time course and the reference value and then estimating the difference between the phase values.

Furthermore, each voxel may be allocated a value that is related to the phase shift between the voxel's time course and a reference. Those signal data in which a new value determined by the methods described above (e.g., correlation, covariance, mutual information analysis, Fourier transform, and etc.) has been allocated to each voxel used is to be understood as modified BOLD signal data.

The region of the brain used to assign a reference value may be determined in several ways. In one variation, one may average the BOLD signal of the brain activity of the entire brain of the patient. In situations of severe damage to brain tissue, an average of the BOLD signal of the entire brain may be misleading, for example, the region of hypoperfusion may be a large proportion of the brain area, which may significantly skew the average BOLD signal. In another variation, one may derive a reference value from less than the entire brain of the patient. Non-limiting examples include, one may use only the left hemisphere of the brain, an arbitrary region of the brain known (e.g., from DWI or otherwise) to be non-affected, a region homologous to the affected region (e.g., if from DWI or otherwise, one suspects that the left motor cortex is affected, one may use the right motor cortex as a reference), and other similar ways.

This may be advantageous since by assigning an estimated time lag value to each voxel of the data specified above it is possible to determine hypoperfused tissue areas with a high degree of precision and which are otherwise detected only in a very unreliable manner by means of PWI.

In addition, the present method is advantageous since no invasive step is necessary for the injection of a contrasting agent that can also cause severe side affects such as but not limited to liver damage, allergies, and others. The method according to the invention is thus more reliable, more precise, and more convenient for the patient than known methods.

Furthermore, the method disclosed herein is advantageous in circumventing the errors inherent in PWI relating to, among other things, the possibility that the change of the arterial blood flow is due to the enrichment with contrasting agent (as opposed to being a symptom of hypoperfusion). So a more precise determination of hypoperfused tissue areas area realised.

In the case of a further advantageous variation the disclosed method may have a further step in which the modified BOLD signal data are sent from the second evaluation device to an imaging device. This is advantageous since the data set (e.g., the set of assigned, pervoxel estimated delay values) are capable of being visualized by the imaging device, so that an optical identification of the area of the affected tissue, in for example the brain, is realised. It is advantageous for this to be carried out by means of calculation in such a way that the BOLD signal data reproduce individual slices of the brain as planar slices.

In a further advantageous embodiment of the method according the invention, the imaging device displays the received modified BOLD signal data preferably by means of a display device as hypoperfused tissue. This is preferred because the display device may by a screen, for example, illustrating the planar slices may be carried out by a display device, such as for example a screen on the fMRI appliance itself, an external screen or even a printer.

In the case of a further advantageous embodiment of the method according to the invention the time delay analysis is applied to at least 50 % of all the voxels, preferably to at least 75 % of all the voxels, and in the most preferred manner to 100 % of all the voxels. This is advantageous since in this way the entire area of the tissue scanned, in this case for example the brain, is detected and a precise determination of the perfusion of the tissue is made possible.

In an additional embodiment the time course of at least one voxel is time-shifted by a ± 15 fold repetition time, preferably by a ± 10 fold repetition time an in particularly preferred manner by a ± 3 fold repetition time. While we refer to the estimated time shift value, an estimate of delay, it should be understood that the phase shift of a voxel in relation to a reference value may be positive, negative, or equal. It is advantageous for a phase shift of this type to be applied to the signal course of each voxel in the chosen area of examination.

It is advantageous for the pre-processing of the BOLD base data to be treated in a further embodiment according to the invention in the first evaluation line by means of algorithms wherein slice-timing, realignment, normalization, removing linear trends and filtering, for example, in the range of from 0.001 to 1 Hz, preferably in the range of from 0.01 to 0.1 Hz is also carried out.

In addition, the application of further algorithms to the BOLD data, which may be magnetic resonance imaging signal data is possible, such as for example establishing the thresholds of the contrast or gradation of the grey tones. It may be advantageous for all the above mentioned algorithms specified to be applied to the BOLD signal data, e.g. magnetic resonance imaging signal data, for pre-processing, wherein the sequence can be freely selected as desired. It is also possible, however, for only part of the algorithms capable of being pre-determined to be selected. This list thus serves only for the purpose of illustration.

In addition, the present invention comprises apparatus for the visualization of hypoperfused, in particular in the case of a acute stroke, for the evaluation of spontaneously occurring local fluctuations of BOLD signal, comprising at least one imaging apparatus for detecting BOLD signal data, a data detection device for receiving the recorded BOLD signals by way of at least one interface, an evaluation device for receiving the BOLD signal data sent by the data detection device, wherein a first evaluation device is provided for the image recognition improvement of the quality of the BOLD data and a second evaluation device is provided for the application of a time delay estimation analysis to the BOLD data of at least one voxel, in order to calculate a time dependent positive and negative time delay of the BOLD data of at least one voxel, in order to determine at least one hypoperfused tissue area by means of correlation between a shifted time course of the at least one voxel reproduced by the phase shift data and the entire average time course during the signal recording for determination purposes. This is advantageous since this apparatus permits the application of the method according to the invention described above.

In addition, it is possible for the apparatus according to the invention to be retrofitted in already known fMRI appliances. It is advantageous for the method according to the invention to be carried out by means of resting state fMRI, so that measurement is facilitated in, for example, patient populations.

In the case of a further advantageous embodiment of the invention the apparatus according to the invention has an imaging device, by means of which the estimated delay value, BOLD signals are capable of being shown.

In addition, in order to use an apparatus to visualize hypoperfused areas, in particular in the event of an acute stroke, to evaluate spontaneously occurring local fluctuations of a BOLD signal comprising at least one BOLD detection apparatus for receiving and recording BOLD data, a data detection device for receiving the recorded BOLD data by way of at least one interface, an evaluation device for receiving the BOLD data sent by the data detection device, wherein a first evaluation device is provided for the image recognition improvement of the quality of the BOLD data and a second evaluation device is provided for the application of a time delay estimation to the BOLD data of at least one voxel, in order to calculate a positive or negative phase delay of the BOLD data of at least one voxel, in order to determine at least one hypoperfused area by means of estimating the temporal delay between the BOLD signal of least one voxel and a reference time course during signal recording for determination purposes, in the event of acute stroke and/or ischaemia.

The use of the method according to the invention and the apparatus according to the invention is of course not limited to brain tissue, but it is capable of being used for any further determination of the tissue inadequately supplied with blood, such as for example in the case of a leg vein thrombosis.

Advantages and expedient properties are evident from the following description in conjunction with the drawing. In the drawing
- Figure 1a: shows an example of a model time course used during the examination;
- Figure 1b: shows a time course of one voxel of the BOLD signal during the examination;
- Figure 2: are examples fMRI images of two patients' brains, and
- Figure 3: is a flow chart of a method according to the invention.

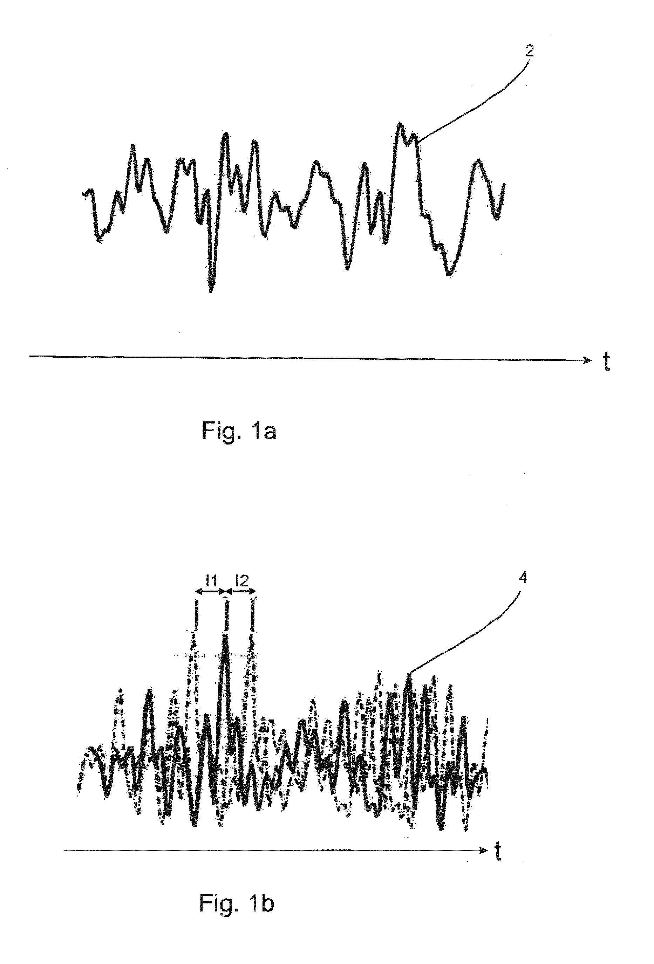

Fig. 1a shows an example time course (2) of the BOLD signal detected during an examination of the patient's brain tissue. In addition, the time course of only one voxel (4) of the BOLD signal is shown in Fig. 1b, the abscissa reproducing the time course and the ordinate the intensity of the signal in both Fig. 1a and Fig. 1b. As a result of applying the time shift analysis to the BOLD signal of one voxel for a time interval I the time course (4) thereof is phase shifted by I1 and by I2 in each case. It is preferable for I1 and I2 totaled to give I. It is advantageous for time values in the range of 15 times the repetition time, preferably of 10 times, and in a most preferable manner 3 times the repetition time, to be selected for I1 and 12.

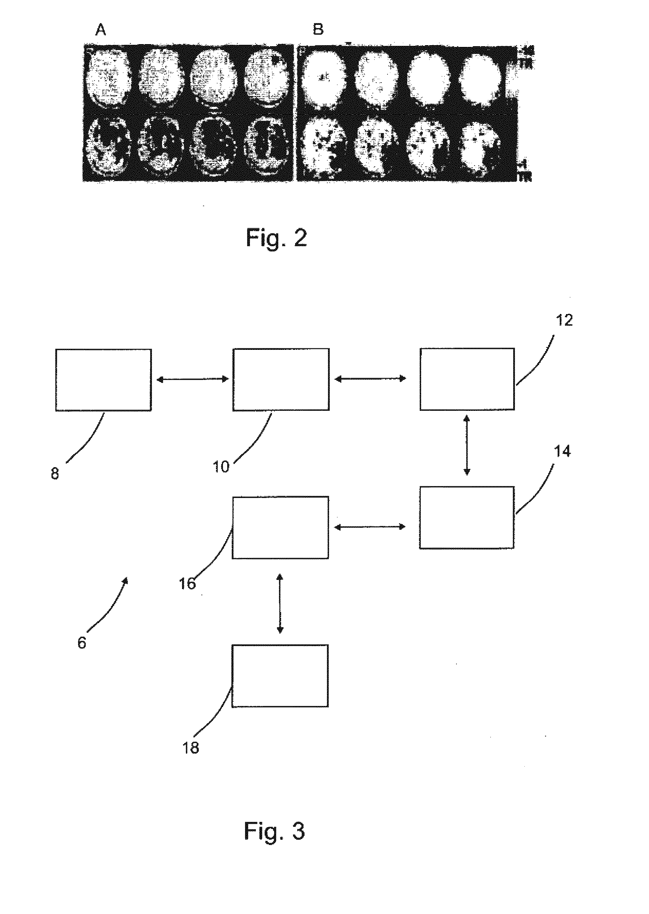

In Fig. 2, fMRI recordings of the brains of two patients (A, B) are shown, which were taken one day after the acute stroke. In this example the data detection was carried out by means of diffusion- and perfusion-weighted imaging, FLAIR and BOLD weighted fMRI recordings (34 planar slice planes; 64 x 64 matrix; voxel dimension of 3 x 3 x 4 mm; flip angle 90°; echo time TE 90 ms; repetition time TR 2300 ms; 150 whole brain EPI volumes). These parameters are selected of course only by way of example and can be selected to be freely variable depending upon the application.

The planar slices of the patients' brains illustrated in the upper row were recorded by means of conventional (contrast enhanced) perfusion MRI showing the less perfused (hypoperfused) area of the tissue. As compared with this, the method according to the invention, by means of which the lower row of recordings was carried out, provides a similar differentiation and localization of the areas of the less perfused brain tissue. This shows that the method according to the invention and the apparatus according to the invention indicate a rapid diagnosis process, by means of which tissue with an inadequate blood supply, in particular in the brain, can be visualized in a rapid and reliable manner, so that it is possible to start the therapy promptly in order to re-establish the blood flow through the brain, so as to prevent permanent damage to the tissue, for example necroses.

Fig. 3 is a diagrammatic flow chart 6. BOLD signal data of the tissue to be examined are detected and recorded by means of a scanning apparatus 8. The recorded BOLD data are passed on to a data detection device 10 that is preferably used as an intermediate store of the data. It is advantageous for the magnetic resonance imaging signal base data to be detected as a BOLD signal.

In a further method step the BOLD data are sent to a first evaluation device 12 or are requested from the data detection device 10.

The pre-processing of the BOLD data is carried out in this first evaluation device 12, so that their quality is improved. In a subsequent method step the pre-processed BOLD are sent to a second evaluation device 14 or are requested from the first evaluation device 12, in order to apply the time delay estimation. After the assignment of a value corresponding to the time delay between a brain region and a reference, data modified in this way are sent to an imaging device 16 which uses the re-calculated voxel in order to produce planar slices of the brain or areas of the tissue examined, it being preferable for the imaging device 16 to be permanently connected to an display device 18. By means of the display device 18 the re-calculated planar slices of the brain are then visualized, for example on a monitor. It is advantageous for the imaging device 16 to have an internal memory, so that the modified BOLD data can be stored permanently.

It is advantageously possible for each of the specified apparatus 8, 18 and of the specified devices 10, 12, 14 and 16 to be designed both in the form of a transmitter and in the form of a receiver, so that the data can be sent and can be received in a passive manner or can also be retrieved in an active manner by a preceding apparatus or device. It is preferably possible for the specified apparatus 8, 18 and the devices 10, 12, 14 and 16 to be at least in part in permanent connection, which can also be regarded as communication. A suitable connection can be carried out for example by means of cables or also in a cable-free manner, for example by means of radio, WLAN or Bluetooth.

The pre-processing of the BOLD data and the time delay estimation can of course also be carried out by an evaluation device, wherein the first and the second evaluation devices together form a single evaluation device.

All the features disclosed in the application documents are claimed as being essential to the invention, insofar as they are novel either individually or in combination as compared with the prior art.

### List of References

- 2: entire signal course of the BOLD signal
- 4: signal course of the BOLD signal of one voxel
- 6: flow chart
- 8: scanning apparatus
- 10: data detection device
- 12: first evaluation device
- 14: second evaluation device
- 16: imaging device
- 18: display device
- I: interval
- I1, I2: phase shift
- TR: repetition time
- TE: echo time

## Claims

1. A method of visualization of areas of the tissue insufficiently supplied with blood, in particular in the event of an acute stroke, by evaluation of local fluctuations of a BOLD signal comprising the following steps:
- producing and recording of BOLD signal data by means of an apparatus (8);
- passing on the BOLD signal data to a data detection device (10) by way of at least one interface;
- sending the BOLD signal data received from the data detection device (10) to an evaluation device;
- pre-processing BOLD in a first evaluation device (12) for image recognition;
- applying a time delay analysis to the BOLD signal data of at least one voxel in a second evaluation device (14) in order to calculate a time dependent positive and negative phase shift (I1, 12) of the BOLD signal data of at least one voxel, and
- assigning a reference value which reference value is an estimated time delay between the BOLD signal data of at least one voxel and the entire average time course of a selected region during the recording of the BOLD data in order to estimate a modified BOLD signal data which are used to determine at least one area of the hypoperfused tissue.

2. A method according to claim 1, **characterized in that** in a further method step the modified BOLD data are sent from the second evaluation device (14) to an imaging device (16).

3. A method according to claim 2, **characterized in that** the imaging device (16) displays the received modified BOLD data preferably by means of a display device (18) as hypoperfused tissue.

4. A method according to any of the preceding claims, **characterized in that** the time delay analysis is applied to at least 50 % of all the voxels, preferably to at least 75 % of all the voxels, and in the most preferred manner to 100 % of all the voxels.

5. A method according to at least one of the preceding claims, **characterized in that** the time course of at least one voxel is time shifted by a ± 15 fold repetition time TR, preferably by a ± 10 fold repetition time TR and in a particularly preferred manner by a ± 3 fold repetition time TR.

6. A method according to claim 1, **characterized in that** in the pre-processing of the BOLD data a filtering in the range of from 0.001 to 1 Hz, preferably from 0.01 to 0.1 Hz, is applied to them.

7. An apparatus for the visualization of hypoperfused, in particular in the case of a acute stroke, for the evaluation of spontaneously occurring local fluctuations of BOLD signal, comprising at least one imaging apparatus (8) for detecting BOLD signal data, a data detection device (10) for receiving the recorded BOLD signals by way of at least one interface, an evaluation device for receiving the BOLD signal data sent by the data detection device, wherein a first evaluation device (12) is provided for the image recognition improvement of the quality of the BOLD data and a second evaluation device (14) is provided for the application of a time delay estimation analysis to the BOLD data of at least one voxel, in order to calculate a time dependent positive and negative time delay of the BOLD data of at least one voxel, in order to determine at least one hypoperfused tissue area by means of correlation between a shifted time course of the at least one voxel reproduced by the phase shift data and the entire average time course during the signal recording for determination purposes.

8. An apparatus according to claim 7, **characterized in that** an additional imaging device (16), by means of which the hypoperfused area is capable of being shown, is provided.

9. Use of an apparatus to visualize hypoperfused areas, in particular in the event of a acute stroke, to evaluate spontaneously occurring local fluctuations of a BOLD signal comprising at least one BOLD detection apparatus (8) for receiving and recording BOLD data, a data detection device (10) for receiving the recorded BOLD data by way of at least one interface, an evaluation device for receiving the BOLD data sent by the data detection device, wherein a first evaluation device (12) is provided for the image recognition improvement of the quality of the BOLD data and a second evaluation device (14) is provided for the application of a time delay estimation to the BOLD data of at least one voxel, in order to calculate a positive or negative phase delay of the BOLD data of at least one voxel, in order to determine at least one hypoperfused area by means of estimating the temporal delay between the BOLD signal of least one voxel and a reference time course during signal recording for determination purposes, in the event of acute stroke and/or ischaemia.
